# EUROPEAN PATENT APPLICATION

(11) **EP 2 221 611 A1**
(43) Date of publication of application: **25.08.2010**
(21) Application number: 10001448.9
(22) Date of filing: 12.02.2010
(51) Int. Cl.: G01N 27/22, G01N 33/03, A47J 37/12

(54) **System for monitoring quality of cooking oil**

(30) Priority: 12.02.2009 US 152045 P
(71) Applicant: Testo AG, 79853 Lenzkirch (DE)
(72) Inventor: Bruinsma, Marko, NJ 07960 Morristown (US); Bove, Robert, NJ 07960 Morristown (US)
(74) Representative: Maucher, Wolfgang

(57) **Abstract**

A system is provided herein for monitoring quality of cooking oil, the system including: at least one sensor for detecting a percentage of total polar materials in cooking oil; at least one transmitter for transmitting the detected percentage; and, a data acquisition system located remotely from the sensor, the data acquisition system including a receiver configured for receiving the transmitted detected percentage. Advantageously, with the subject invention, a system may be provided which allows for large scale monitoring of cooking oil at multiple locations.

## Description

### BACKGROUND OF THE INVENTION:

Devices are known in the prior art for measuring quality of cooking oil. For example, with reference to U.S. Patent No. 6,822,461, a sensor is known for taking capacitive measurements of cooking oil to evaluate the change in dielectric constant which is an indicator of the amount of polar components in the cooking oil. Cooking oil, through use, oxidizes with a resultant accumulation of polar components. An excessive amount of polar components results in poor food taste and, even possible health risks. As a result, the level of polar components, referred to as the percentage of total polar materials (TPM), is an indicator of the quality of the cooking oil.

Although such a sensor is known in the prior art, monitoring quality of cooking oil at industrial levels, particularly at multiple locations and/or multiple cooking units, has not been practicably handled in the prior art. Replacement of cooking oil prematurely results in waste, while cooking oil used excessively results in poor quality and health risks. Industrial management of cooking oil is desired to allow for efficient replacement thereof.

### SUMMARY OF THE INVENTION:

A system is provided herein for monitoring quality of cooking oil, the system including: at least one sensor for detecting a percentage of total polar materials in cooking oil; at least one transmitter for transmitting the detected percentage; and, a data acquisition system located remotely from the sensor, the data acquisition system including a receiver configured for receiving the transmitted detected percentage. Advantageously, with the subject invention, a system may be provided which allows for large scale monitoring of cooking oil at multiple locations.

These and other features of the invention will be better understood through a study of the following detailed description and accompanying drawings.

### BRIEF DESCRIPTION OF THE DRAWINGS:

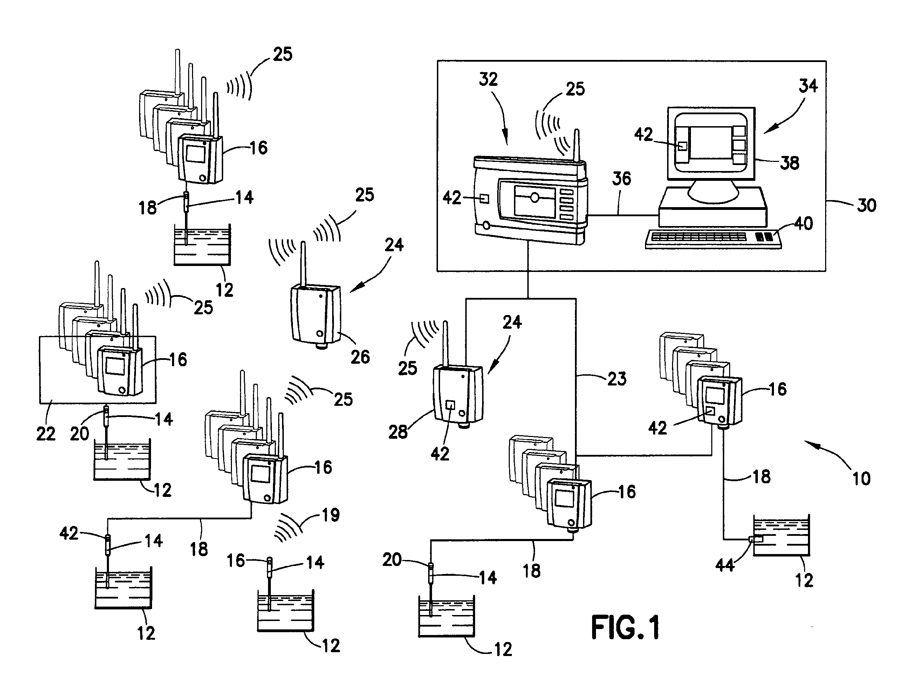

Fig. 1 is a schematic of a system formed in accordance with the subject invention.

### DETAILED DESCRIPTION OF THE INVENTION:

A system 10 is provided herein for monitoring the quality of cooking oil 12. The cooking oil 12 is monitored during use, where the cooking oil 12 is subjected to elevated temperatures resulting in oxidation reaction. As a result of the oxidation, the level of total polar materials (TPM) increases. An excessively high level of the total polar materials, which can be gauged as a percentage of total polar materials relative to the total amount of cooking oil, may lead to poor quality and possible health risks. The system 10 may be used to monitor the quality of the cooking oil 12 to provide an indication as to when the cooking oil 12 should be changed. Premature changing of the cooking oil 12 may result in inefficient and wasteful spending, whereas an excessively-used batch of the cooking oil 12 may result in the poor quality and health risks discussed above.

As will be appreciated by those skilled in the art, the cooking oil 12 may be of any type, including, but not limited to, rapeseed oil, peanut oil, coconut oil and combinations thereof.

The system 10 includes one or more sensors 14 for detecting the percentage of total polar materials in the cooking oil 12. The sensors 14 may be of any known type. Preferably, the sensors 14 are of the capacitive type disclosed in U.S. Patent No. 6,822,461, the entire contents of which are incorporated by reference herein. In particular, U.S. Patent No. 6,822,461 discloses a capacitive sensor for detecting the level of the total polar materials. The sensor includes a temperature gauge for measuring the temperature of the cooking oil during testing. The temperature of the cooking oil is used to adjust the measured capacitance such that a more correct reading is achieved. This adjusted reading is then used to determine the total polar materials content of a test sample. The sensors 14 herein may be also configured to measure the temperature of the cooking oil 12.

One or more transmitters 16 are provided for the sensors 14. The transmitters 16 are configured to transmit data relating to detected levels of the total polar materials measured by the sensors 14. The transmitters 16 may be hard-wired to the sensors 14, such as through a hard-wired electrical connection 18, or wirelessly coupled with the sensors 14, through signal 19, which may be of any one-way or two-way wireless signal, such as radio or cellular. The transmitters 16 may be provided separately from the sensors 14 and/or coupled therewith, such as being housed within the sensors 14. In this manner, any measured data may be instantaneously transmitted by the transmitter 16. In case of power failure or other disruption, a local memory 20, such as a flash drive or EEPROM, may be provided for the sensors 14 to store a limited number of test results. The local memory 20 may be located directly on the sensors 14. In addition, or alternatively, a cradle or other docking device 22 may be provided in which the sensor 14 is mounted after use. The cradle 22 may contain the transmitter 16. A jack or other connection configuration may be provided on the sensor 14 and the cradle 22 to permit direct connection between the sensor 14 and the transmitter 16. This configuration would require the use of a local memory 20 since any measured data must be retained on the sensor 14 until the sensor 14 is caused to dock into the cradle 22, thus permitting connection with the transmitter 16. As a further alternative, the transmitter 16 may be formed directly onto the sensor 14. The sensors 14 may be provided with displays to permit real-time display on test results.

As shown in Fig. 1, the system 10 may include any number of the sensors 14 and the transmitters 16, depending on the number of batches of the cooking oil 12 to be monitored. The cooking oil 12 may be at various locations, including being at substantial distances apart, for example, in different restaurant locations in different cities. The multiple locations of the cooking oil 12 may also be at a common location but spread apart in a facility, such as in a large-scale industrial baking facility. The transmitters 16 permit data obtained at each location of the cooking oil 12 to be transmitted to a central location for evaluation.

At least one receiver 24 is provided which is configured to receive the data transmitted by one or more of the transmitters 16, particularly the transmitted percentages of total polar materials. The transmitters 16 and the receivers 24 may be linked together using any known configuration, including a network configuration 23, such as an Ethernet, large area network (LAN) or wide area network (WAN) and so forth, or through signals 25 transmitted by wireless communication, such as using radio or cellular technology. The receivers 24 may be configured to include additional features. Where multiple wireless connections between the transmitters 16 and the receivers 24 are used, the receiver 24 may also include a router 26 configured to intercept and queue the wireless signals 25 from a plurality of the transmitters 16 prior to further processing. The router 26 may be further configured to transmit the received signals 25 in an orderly sequence for further processing. In addition, the receiver 24 may include a converter 28 utilized to intercept the wireless signals 25, such as radio signals, with conversion to a signal suitable for transmission over the network 23, such as an Ethernet. The receivers 24 may be configured with both the router 26 and/or the converter 28 feature(s).

A data acquisition system 30 may be provided which includes a master receiver 32 for receiving signals from all of the transmitters 16. The signals may have been transmitted via the receivers 24 and been processed by the router 26 and/or the converter 28. In addition, the master receiver 32 may receive signals directly from some or all of the transmitters 16. The master receiver 32 may be provided with the router 26 and/or the converter 28 feature(s).

A CPU or other computing device 34 may be provided which is linked through a connection 36, e.g., a USB cable, to the master receiver 32. Data received by the master receiver 32 is conveyed to the CPU 34 for monitoring and/or analysis. The CPU may store collected data. A display 38 may be provided which allows for the various levels of total polar materials measured by the sensors 14 at different locations to be displayed (e.g., graphical or tabular display). In addition, a user interface 40, such as a keyboard, a mouse, a touch pad, a touch screen and so forth, may be used in conjunction with the CPU 34 to manipulate the accumulated data and to optionally control various components of the system 10. To allow for the control of the system 10, the transmitters 16, the receivers 24, and the master receiver 32 may be configured to be two-way devices, configured to both transmit and receive messages. Through the use of the user interface 40, parameters at the sensors 14 may be adjusted, such as temperature correction parameters, which for example may be used in correcting measured capacitance. In addition, the user interface 40 may be used to alter the parameters to adjust to a different type of the cooking oil 12, for example in adjusting for a change from peanut oil to coconut oil. The user interface 40 may be configured to directly alter parameters or other features located on the local memory 20 of the sensors 14.

Preferably, the transmitters 16, the receivers 24, and the master receiver 32 use two-way signal transmission and reception to minimize cross-talk errors, transmission errors, and for security concerns. The two-way signal transmission permits a signal "hand shake" to be defined prior to, and/or periodically during, data transmission.

An alarm 42 may be located at one or more locations in the system 10 which provides an indication that a predetermined threshold amount of the total polar materials has been exceeded. The alarm 42 may be located at one or more of the sensors 14, the transmitters 16, the receivers 24, the master receiver 32, and/or the CPU 34. The alarm 42 may be any combination of an audible alarm, a visual alarm and/or an electronic notification, such as an e-mail or SMS text message. The alarm 42 provides an indication that an identified batch of the cooking oil 12 should be replaced.

As will be appreciated by those skilled in the art, the sensors 14 may be hand-held, portable devices or may be configured to be submersed devices 44 in the cooking oil 12. The sensors 12 may require periodic maintenance and calibration.

The data acquisition system 30 is located to be remote from the sensors 14. As used herein, the term "remote" and derivatives thereof, indicates at a distance greater than where an individual can both use the sensor 14 in a batch of the cooking oil 12 and engage the interface 40. In other words, the data acquisition system 30 requires an individual to travel from one or more of the batches of the cooking oil 12 to get to the interface 40. The duration of travel may vary from several paces, to travel to another building or city.

With the data acquisition system 30 being located remotely from the sensors 14, data can be centralized for observation and analysis. With the sensors 14 measuring temperature of the cooking oil 12, the temperatures may be collected by the data acquisition system 30. In addition, the system 10 can be configured to be coupled to other instruments, test equipment, monitoring equipment, counting equipment, and so forth, to collect data in similar fashion. For example, door sensors may be applied to freezers to count the number of door openings over a period of time. Inventory can likewise be monitored.

## Claims

1. A system for monitoring quality of cooking oil, said system comprising:
at least one sensor for detecting a percentage of total polar materials in cooking oil;
at least one transmitter for transmitting said detected percentage; and,
a data acquisition system located remotely from said sensor, said data acquisition system including a receiver configured for receiving said transmitted detected percentage.

2. A system as in claim 1, wherein said transmitter is located on said sensor.

3. A system as in claim 1, wherein said transmitter is a wireless transmitter and said receiver is a wireless receiver.

4. A system as in claim 1, wherein said transmitter is electrically hard-wired coupled to said receiver.

5. A system as in claim 1, wherein said data acquisition system further includes a display.

6. A system as in claim 5, wherein said transmitted detected percentage is displayable on said display.

7. A system as in claim 1, wherein said data acquisition system further includes memory for storing a plurality of said transmitted detected percentages.

8. A system as in claim 1, wherein said data acquisition system further includes a user interface which permits a user to selectively control said data acquisition system.

9. A system as in claim 8, wherein said user interface may be used to selectively control the system.

10. A system as in claim 1, further comprising an alarm which is activated with said detected percentage being over a pre-determined threshold value.

11. A system as in claim 1, further comprising an alarm which is activated with said transmitted detected percentage being over a pre-determined threshold value.

12. A system as in claim 1, wherein said sensor is configured to measure temperature of said cooking oil.

13. A system as in claim 12, wherein said measured temperature is transmittable by said transmitter and receivable by said receiver.

14. A system as in claim 1, wherein said transmitter and said receiver are configured to transmit therebetween one or more signals which confirm proper communication between said transmitter and said receiver.

15. A system as in claim 1, wherein said sensor includes a local memory, and wherein, parameters usable in detecting said detected percentage are storable in said local memory.

16. A system as in claim 15, wherein said parameters may be adjusted by said data acquisition system via said receiver and said transmitter.

17. A system as in claim 16, wherein said parameters include temperature correction parameters.

18. A system as in claim 16, wherein one or more set of parameters is provided, each said set corresponding to a different type of cooking oil.
